Europäisches Patentamt

European Patent Office

Office européen des brevets

(19)

(11) EP 1 486 184 A1

(12) EUROPEAN PATENT APPLICATION

(43) Date of publication:
15.12.2004 Bulletin 2004/51

(51) Int Cl.⁷: **A61F 5/455**, A61F 5/44,
A61F 13/15

(21) Application number: 04013652.5

(22) Date of filing: 09.06.2004

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **11.06.2003  JP  2003166671**

(71) Applicants:
• **Hitachi, Ltd.**
  **Chiyoda-ku, Tokyo 101-8010 (JP)**
• **Uni-Charm Corporation**
  **Shikokuchuo-shi Ehime 799-0111 (JP)**

(72) Inventors:
• **Okabe, Kenichi**
  **Niihari-gun Ibaraki 315-0054 (JP)**
• **Kobayashi, Junichi**
  **Ushiku-shi Ibaraki 300-1237 (JP)**

• **Machida, Shigeru**
  **Nishiibaraki-gun Ibaraki 319-0208 (JP)**
• **Miyagawa, Ryousuke**
  **Kasukabe-shi Saitama 344-0032 (JP)**
• **Wada, Ichiro Uni-Charm Corporation**
  **Toyohama-cho, Mitoyo-gun**
  **Kagawa 769-1602 (JP)**
• **Taniguchi, Hiroaki Uni-Charm Corporation**
  **Toyohama-cho, Mitoyo-gun**
  **Kagawa 769-1602 (JP)**
• **Suzuki, Miou Uni-Charm Corporation**
  **Toyohama-cho, Mitoyo-gun**
  **Kagawa 769-1602 (JP)**

(74) Representative: **Beetz & Partner Patentanwälte**
**Steinsdorfstrasse 10**
**80538 München (DE)**

(54) **Automatic urine disposal device and urine receptacle used therefor**

(57)   An automatic urine disposal device comprises a urine receptacle (1) worn by a wearer and a vacuum pump (23) for absorbing urine collected in the receptacle by means of a suction force. The urine receptacle comprises a perforated urine absorbent sheet (3) for absorbing urine discharged in a top sheet (2), and a support sheet (5) in which a urine absorbing space forming sheet (4) is interposed. The urine receptacle is connected to a sealed urine tank (21) via a urine drainage tube (10). The vacuum pump absorbs air from the urine tank to draw urine from the urine receptacle to the urine tank.

## FIG. 5

EP 1 486 184 A1

**Description**

BACKGROUND OF THE INVENTION

The present invention relates to an automatic urine disposal device worn by the bedridden elderly, hospitalized patients, physically disabled people, and others who are unable to voluntarily control the bladder or to clean up urine on their own and also relates to a urine receptacle used therefor.

Because of age, physical disability, hospitalization due to injury or illness, or other physical conditions, people sometimes become unable to voluntarily control the bladder or clean up urine on their own. In those situations, generally, a catheter is directly inserted into the bladder to discharge urine or paper diaper is used.

When a catheter is directly inserted into the bladder, the wearer feels great discomfort and there is also the probability of injuring the urethra or bladder or the occurrence of an infection. Thus, expertise as well as special sterilized utensils is required.

When a paper diaper is worn for a prolonged period of time, urine may leak, the wearer can become uncomfortable, get stuffy, or skin troubles such as rashes may occur. To avoid this, the paper diaper must be frequently changed, which will impose considerable physical and mental burdens on both the wearer and the caretaker. Imposed on a daily basis, those physical and mental burdens become a big concern and a significant economical burden as well.

To avoid those problems, a method has been presented in which urine that has been absorbed by a urine absorbent material of a urine receptacle is discharged by means of a vacuum pump and drawn into a urine tank. The vacuum pump absorbs air in a sealed urine tank and due to the difference between the tank's pressure and the atmospheric pressure, urine absorbed in the urine absorbent material is drained into the urine tank. Automatic urine disposal devices of such configuration have been disclosed in Japanese Application Patent Laid-open Publication No. Hei 07-171182 and No. Hei 11-113946.

In the prior art disclosed in Japanese Application Patent Laid-open Publication No. Hei 07-171182 and No. Hei 11-113946, urine is drained from one location of the urine absorbent material, and therefore, the percentage of urine collection from the urine receptacle (urine absorbent material) is low. For this reason, the amount of urine which remains in the urine receptacle (urine absorbent material) is large, which makes the wearer feel uncomfortable. To reduce the amount of urine which remains in the urine receptacle, the capacity of the vacuum pump must be increased. Accordingly, it becomes necessary to increase the size and volume of the urine disposal device.

BRIEF SUMMARY OF THE INVENTION

An object of the present invention is to provide a compact and lightweight automatic urine disposal device which increases the percentage of urine collection by the urine receptacle and also to provide a urine receptacle used therefor.

To achieve the aforementioned object, the present invention is designed such that it incorporates a urine receptacle in which an urine absorbing space forming sheet is disposed between a perforated urine absorbent sheet for absorbing urine discharged in a top sheet and a support sheet, and urine is drawn through a urine drainage port formed on the support sheet into a sealed urine tank by means of a vacuum pump via a urine drainage tube.

In other words, in the present invention, a urine absorbent layer is formed by using a urine absorbing space forming sheet disposed between a perforated urine absorbent sheet on which a liquid-permeable, hard-breathable top sheet is located and a non-breathable, liquid-impermeable support sheet, and urine is drained by making the pressure in a large number of urine drainage pores formed on the perforated urine absorbent sheet negative, and then urine is drawn into the urine tank via a urine drainage tube.

In the urine receptacle used for the present invention, urine which has been discharged in the top sheet is absorbed through a large number of urine drainage pores formed on the perforated urine absorbent sheet. Therefore, the percentage of urine collection from the urine receptacle increases and the amount of urine which remains in the urine receptacle is reduced. With the increase in the percentage of urine collection, a small-capacity vacuum pump with a low suction force can drain urine. Therefore, it is possible to drain urine from the urine receptacle without discomfort to the wearer, and the device can be compact and lightweight.

BRIEF DESCRIPTION OF THE SEVERAL VIEWS OF THE DRAWING

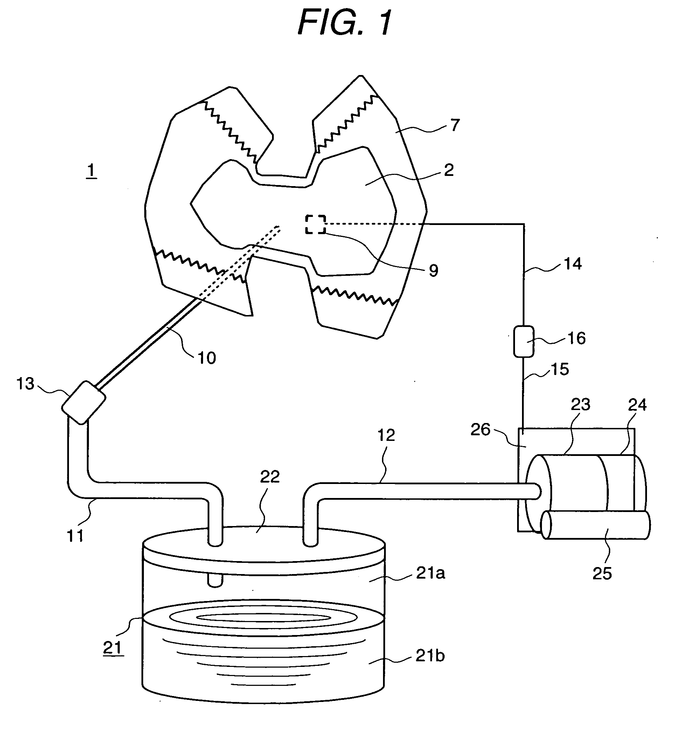

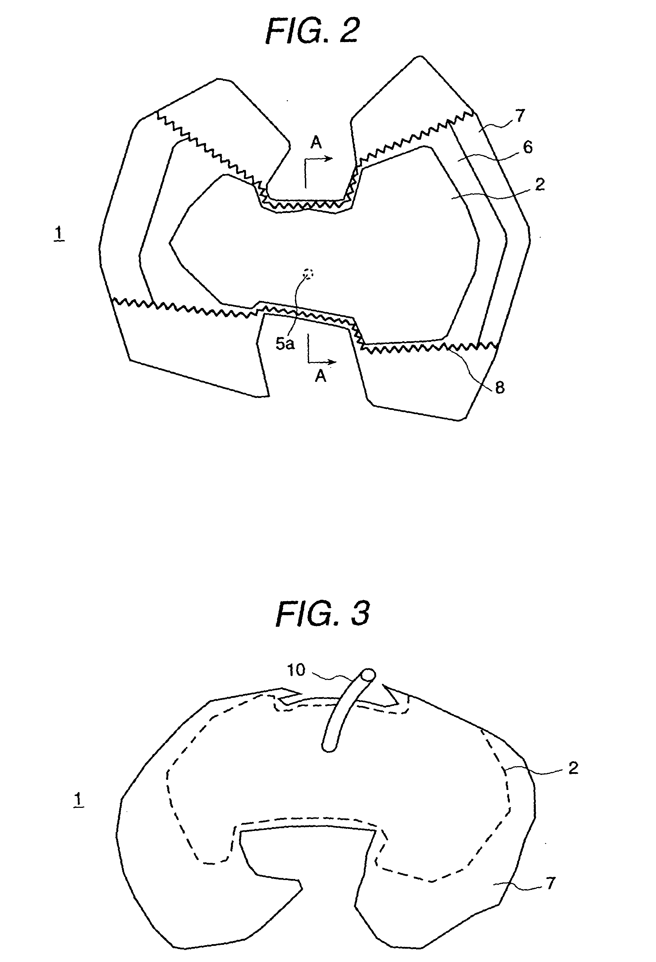

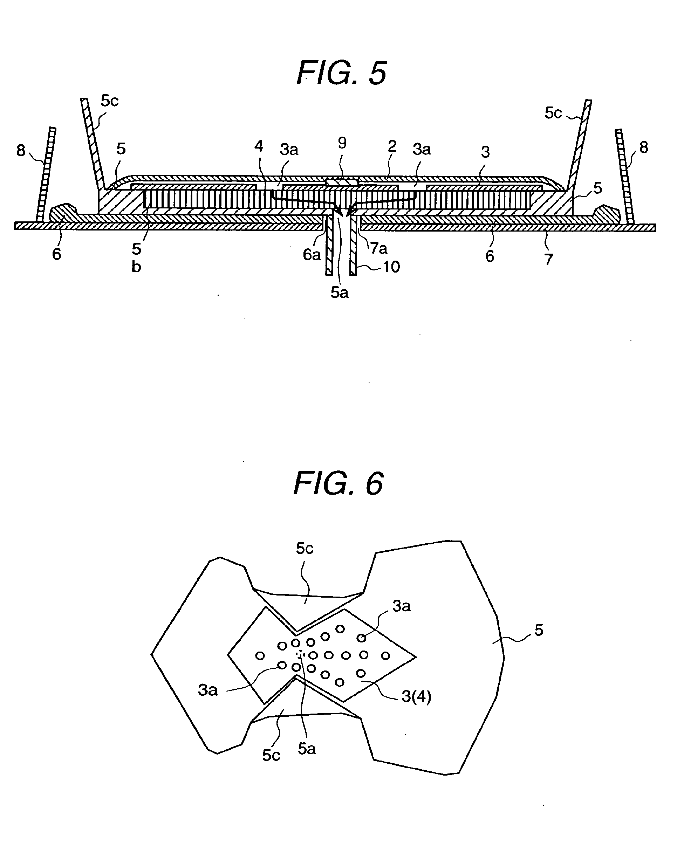

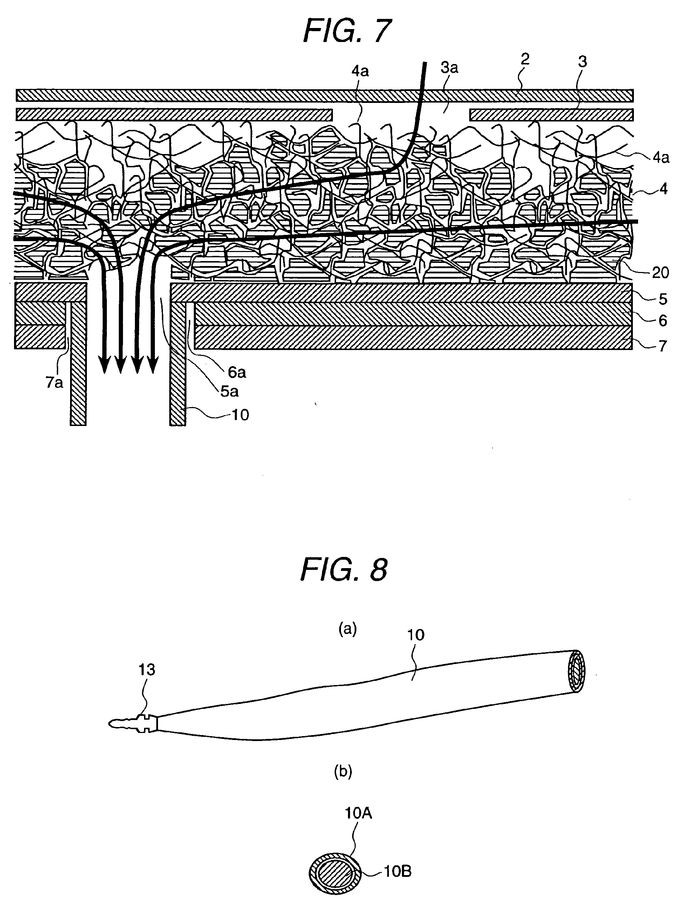

FIG. 1 is a schematic diagram of an embodiment of the present invention, and FIG. 2 through FIG. 4 are a top view, a bottom view, and an exploded perspective view of the urine receptacle, respectively. FIG. 5 is a cross-sectional view taken along the line A-A in FIG. 2. FIG. 6 is an enlarged plan view of a support sheet which is a component of the urine receptacle. FIG. 7 explains functions of the urine receptacle, and FIG. 8 shows another embodiment of a urine drainage tube.

DETAILED DESCRIPTION OF THE INVENTION

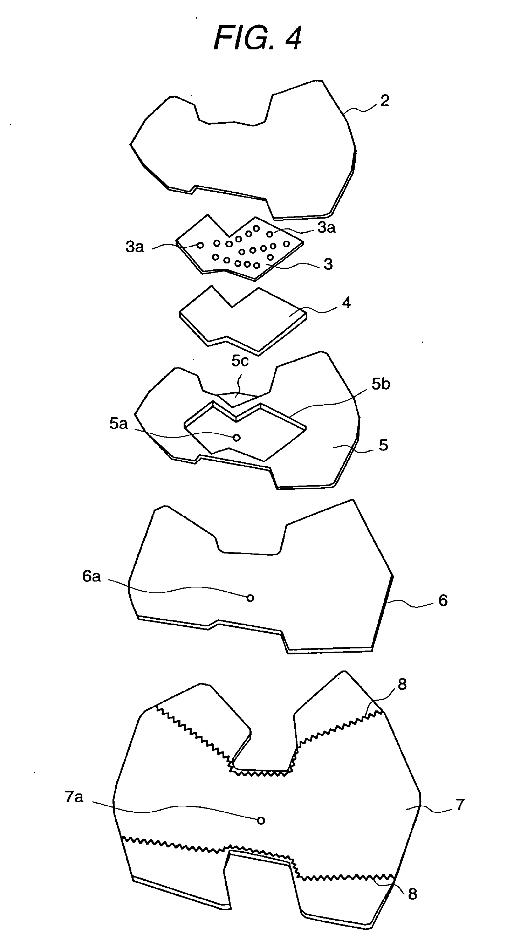

FIG. 1 through FIG. 6 show an embodiment of the present invention. FIG. 1 is a schematic diagram of an embodiment of the present invention. FIG. 2 is a top view of the urine receptacle, FIG. 3 is a bottom view of the urine receptacle, and FIG. 4 is an exploded perspective view of the urine receptacle. FIG. 5 is an enlarged cross-sectional view taken along the line A-A in FIG. 2. FIG. 6 is an enlarged plan view of a support sheet which is a component of the urine receptacle.

In Figures 1 through 6, a urine receptacle 1 which absorbs urine discharged from a wearer's urinating part, not shown, is in the concaved shape, as shown in FIG. 3, and its width at the middle portion in the longitudinal direction (direction of the wearer's front and rear) 2 is narrow so that it is shaped like an hourglass. The reason for this shape is to fit the wearer's crotch.

As shown in FIG. 4, the urine receptacle 1 consists of a top sheet 2, perforated urine absorbent sheet 3, urine absorbing space forming sheet 4, support sheet 5, urine absorbent sheet 6, outer sheet 7 and gathers 8. The top sheet 2 is made of a liquid-permeable, hard-breathable nonwoven cloth, and the perforated urine absorbent sheet 3 is made of liquid-impermeable, non-breathable vinyl. The urine absorbing space forming sheet 4 is made of a rough fibrous material which does not hold liquid, and the support sheet 5 is made of a liquid-impermeable, non-breathable, elastic polyethylene sheet, rubber, or vinyl. Furthermore, the urine absorbent sheet 6 is made of a compound material which compounds a liquid-permeable nonwoven cloth, flocculent pulp, and a polymer absorbent, and the outer sheet 7 and gathers 8 are made of liquid-impermeable nonwoven cloths.

The outer sheet 7 is a thin, liquid-impermeable sheet and is made of a polyethylene film, for example. The outer sheet 7 may be simply made of a liquid-impermeable member; however, desirably, an optimal member can be chosen by taking into account stuffiness which may result from the prolonged use. The outer surface of the outer sheet 7 is laminated with a soft and smooth surface material (not shown), such as a polypropylene nonwoven cloth, to prevent the wearer from becoming uncomfortable. The inner surface of the outer sheet 7 has been treated with a water-repellent material.

Along the periphery of the outer sheet 7 in the longitudinal direction (direction of the wearer's front and rear), three-dimensional gathers 8 are created such that they are slanted inwardly along the periphery of the outer sheet 7 as shown in FIG. 5. These slantingly provided three-dimensional gathers 8 prevent leaks from the sides caused by the wearer's physical activity or change of posture. In addition, a through hole 7a through which a urine drainage tube 10 passes is created on the outer sheet 7.

A urine absorbent sheet 6 adheres to the top surface of the outer sheet 7. The urine absorbent sheet 6 is provided to absorb urine which has not been collected by the support sheet 5, thereby preventing urine from wetting the wearer's clothes or bedding and also preventing urine from coming in contact with the wearer's skin. A through hole 6a through which a urine drainage tube 10 passes is created on the urine absorbent sheet 6.

A support sheet 5 adheres to the surface of the urine absorbent sheet 6. The support sheet 5 has a concave portion 5b into which the urine absorbing space forming sheet 4 can fit. Furthermore, a urine drainage port 5a to which a urine drainage tube 10 is connected is formed in the concave portion 5b. The urine drainage port 5a is formed such that it is located at an approximate center of the perforated urine absorbent sheet 3, which will be described later, on which a large number of urine drainage pores 3a are formed.

Both sides of the support sheet 5 which fit to the wearer's crotch (urinating part) are loose fitting thereby forming peripheral pieces 5c, as shown in Figures 5 and 6, to prevent urine from leaking from the sides. In FIG. 6, a urine absorbing space forming sheet 4 fits into the concave portion 5b, and a perforated urine absorbent sheet 3 is disposed on the top surface of the urine absorbing space forming sheet 4. The support sheet 5 is made of an elastic material such as polyethylene foam.

The urine absorbing space forming sheet 4 is made of a porous, fibriform skeletal material with no water-absorption capability and the sides of the sheet 4 adhere to the concave portion 5b of the support sheet 5 thereby molding into it. The urine absorbing space forming sheet 4 is made of a 5 to 10 mm thick, porous material to ensure that the air space for sucking urine (urine collecting space) is maximized. The urine absorbing space forming sheet 4 fits into the concave portion 5b of the support sheet 5. This configuration eliminates clearance or level difference between the urine absorbing space forming sheet 4 and the support sheet 5 thereby preventing urine from remaining.

A perforated urine absorbent sheet 3 on which a large number of urine drainage pores 3a are formed is disposed on the top surface of the urine absorbing space forming sheet 4. The urine absorbing space forming sheet 4 is disposed such that it comes in close contact with the bottom surface of the perforated urine absorbent sheet 3. The perforated urine absorbent sheet 3 is a similar shape as the urine absorbing space forming sheet 4, and the bottom surface of its periphery adheres to the support sheet 5 as shown in FIG. 5. Thus, the urine absorbing space forming sheet 4 is disposed between the perforated urine absorbent sheet 3 and the support sheet 5.

Urine drainage pores 3a are formed on the perforated urine absorbent sheet 3 between the area where urine is discharged from the urinating part and the vicinity of the buttocks in order to increase the percentage of urine collection as well as to handle spread of urine due to urination while the wearer is lying down on his/her back or sitting. Urine drainage pores 3a are formed at locations with different distances necessary for effectively collecting urine. Furthermore, the diameter of the urine drainage pore 3a can be changed according to its location from which urine is collected; for example, the diameter of the pore near the urinating part can be made larger than that of other pores.

The top sheet 2 is disposed on the surface of the perforated urine absorbent sheet 3 and a urine sensor 9 is located between the top sheet 2 and the perforated urine absorbent sheet 3. The surface of the perforated urine absorbent sheet 3 is covered with the hard-breathable top sheet 2. Urine discharged by a wearer is absorbed by the top sheet 2 and then absorbed into urine drainage pores 3a formed on the perforated urine absorbent sheet 3. The top sheet 2 is made of a liquid-permeable, hard-breathable nonwoven cloth which is made of, for example, polypropylene and polyolefin polyester blended with cotton so that friction between the wearer's skin and the fabric is minimized.

In addition, a mesh sheet makes up a part of the surface of the nonwoven cloth used as the top sheet 2 where it comes in contact with the wearer's urinating part and the surrounding skin. This is to increase the liquid-absorbent and sweat-absorbent capabilities so that urine can be quickly absorbed by the urine drainage pores 3a formed on the perforated urine absorbent sheet 3 through small pores created in the mesh sheet. Because urine can be quickly absorbed by the perforated urine absorbent sheet 3, the wearer has a minimal amount of discomfort due to moisture around the wearer's urinating part.

Herein, hard breathability of the top sheet 2 will be explained. Hard breathability of the top sheet 2 means that the breathability measured according to the General Textile Testing Method's breathability testing method A, prescribed in JIS L1096, 6.27.1, is from 0 to 100 $cc/cm^2/second$ and preferably from 0 to 50 $cc/cm^2/second$ when the top sheet 2 is moist. When the top sheet 2 is dry, the breathability is from 20 to 200 $cc/cm^2/second$, preferably from 20 to 100 $cc/cm^2/second$, and more preferably from 20 to 50 $cc/cm^2/second$.

Moreover, "being moist" is a condition in which moisture content (%) of the top sheet 2 that is obtained by the following equation is 100 % or more, and "being dry" is a condition in which the top sheet 2 has been left dry in the 20 °C and RH60 % atmosphere, or the condition of, what is called, official moisture regain.

$$\text{Moisture content} = (\text{Weight of moist sheet - Weight of dry sheet})/(\text{Weight of dry sheet}) \qquad \text{(Equation 1)}$$

In FIG. 1, one end of the urine drainage tube (first urine drainage means) 10 is connected to the urine drainage port 5a formed on the support sheet 5. The urine drainage tube 10 penetrates holes 6a and 7a of the urine absorbent sheet 6 and the outer sheet 7 and the other end is connected to a one-touch joint 13. The one-touch joint 13 is mounted to one end of the urine drainage tube (second urine drainage means) 11. This joint 13 connects the other end of the urine drainage tube 10 to the end of the urine drainage tube 11. The urine drainage tubes 10 and 11 are made of soft, flexible materials such as soft resin, and the one-touch joint 13 is made of a soft material.

A urine tank 21 is sealed by a lid 22. The other end of the urine drainage tube 11 passes through the lid 22 of the urine tank 21 and is located in the vapor phase area 21a of the urine tank 21. One end of the vacuum tube 12 is connected to a vacuum pump 23 and the other end passes through the lid 22 of the urine tank 21 and is located in the vapor phase area 21a of the urine tank 21. Like the urine drainage tubes 10 and 11, the vacuum tube 12 is also made of a soft, flexible material. The capacity of the urine tank 21 is about 500 $cm^3$ which can store two separate urinations. This tank also comes in 200 $cm^3$ or 1000 $cm^3$ which allows for the prolonged use at night.

The vacuum pump 23 is driven by a motor 24. The motor 24 uses a battery 25 as a driving power source, and is controlled by a control device installed in the control board 26. The vacuum pump 23 is small having a diameter of 30 mm × 70 mm. Voltage of the battery 25 is approximately 6 V.

A urine sensor 9 detects that urine has been discharged in the top sheet 2. It is electrically conductive and detects the wearer's urination by sensing the resistance value change. The urine detection signal detected by the urine sensor 9 is inputted via signal lines 14 and 15 into the control board 26 that controls the vacuum pump 23. The signal lines 14 and 15 are connected by a one-touch joint 16.

In this configuration, the urine receptacle 1 is worn so that the top sheet 2 comes in contact with the urinating part of a wearer (not shown) in his/her underwear. The urine tank 21, vacuum pump 23, and the motor 24 can be carried by the wearer or can be placed on or under the bed on which the wearer lies.

When the wearer urinates in this situation, urine discharged in the urine receptacle 1 is absorbed by the top sheet (nonwoven cloth) 2. When the top sheet 2 absorbs urine, the space among fibers of the top sheet 2 is filled with urine. When the top sheet 2 absorbs urine, the urine sensor 9 is turned on, and a urine detection signal is inputted into the control board 26. The control device installed in the control board 26 activates the motor 24 to drive the vacuum pump 23.

When air in the urine tank 21 has been discharged by the vacuum pump 23, air pressure in the urine absorbing space inside the urine absorbing space forming sheet 4 decreases, creating negative pressure in a large number of urine drainage pores 3a formed on the perforated urine absorbent sheet 3. The top sheet 2 is hard-breathable and the outer sheet 7 is non-breathable. Therefore, pressure in the urine absorbing space of the urine absorbing space forming sheet 4 efficiently decreases.

When pressure in the urine absorbing space of the urine absorbing space forming sheet 4 becomes negative, a uniform suction force is created in all of the urine drainage pores 3a. Consequently, it is possible to draw urine from a urine drainage pore 3a away from the urine drainage port 5a to the urine absorbing space. As shown in FIG. 7, urine 20 (indicated by horizontally hatched lines) flows into the pores of the fibrous material 4a which create a urine absorbing space 4A of the urine absorbing space forming sheet 4 and flows in the direction indicated by thick arrows. Urine absorbed by the urine absorbing space forming sheet 4 is drawn into a urine drainage tube 10 by means of a suction force due to negative pressure.

The urine drainage pores 3a of the perforated urine absorbent sheet 3 are formed in such a way that, with the appropriate number of pores of a certain diameter, the urine drainage pores 3a properly maintain negative pressure in the urine absorbing space so as to draw urine into the urine absorbing space by means of a uniform suction force from a wide area of the top sheet 2 which comes in contact with the perforated urine absorbent sheet 3. That is, the perforated urine absorbent sheet 3 is provided in order to uniformly create negative pressure in the urine absorbing space of the urine absorbing space forming sheet 4. Without the perforated urine absorbent sheet 3, urine is exclusively drawn from the vicinity of the urine drainage port 5a (urine drainage tube 10) into the urine drainage tube 10, causing the percentage of urine collection to decrease.

Urine drawn into the urine drainage tube 10 is further drawn into the urine tank 21 by the negative pressure via the urine drainage tube 11 and stored in the tank. The urine tank 21 can be removed by disconnecting the urine drainage tubes 10 and 11 by unlocking the one-touch joint 13 as well as by disconnecting the signal lines 14 and 15 by unlocking the one-touch joint 16. Thus, the automatic urine disposal device including the urine tank 21 can be carried and urine stored therein can be disposed of. The urine tank 21 can be carried by removing the lid 22 from the urine tank 21 and urine stored in the urine tank 21 can be disposed of.

Moreover, after the wearer has worn the urine receptacle 1 for a day or when it became dirty due to defecation, the used urine receptacle 1 is discarded by disconnecting the urine drainage tube 10 from the urine sensor 9 by unlocking the joints 13 and 16, and the wearer wears a new urine receptacle 1.

A urine receptacle 1 according to the present invention is designed such that a urine absorbing space forming sheet 4 is disposed between a perforated urine absorbent sheet 3 and a support sheet 5, and urine is drained through a large number of urine drainage pores 3a formed on the perforated urine absorbent sheet 3 and is drawn into a sealed urine tank 21 by means of a vacuum pump 23 via urine drainage tubes 10 and 11. This configuration makes it possible to maintain high percentage of urine collection even if the vacuum pump 23 has a low suction force.

FIG. 8 shows another embodiment of a urine drainage tube 10 which is connected to the urine receptacle 1. The urine drainage tube 10 shown in FIG. 8 is different from the urine drainage tube in the above-mentioned embodiment. In FIG. 8, a space-holding material 10B is provided on the inner surface of the outer shell 10A of the urine drainage tube 10. This configuration allows the outer shell 10A to be made of a soft material such as liquid-impermeable vinyl and to ensure a space for vacuuming urine without being compressed by a wearer's weight. Furthermore, in the aforementioned embodiment, a soft flexible material, such as liquid-impermeable rubber or soft resin, must have sufficient rigidity so that it will not be compressed by a wearer's weight. However, the urine drainage tube 10 shown in FIG. 8 can have more flexibility thereby reducing discomfort to the wearer.

As state above, in the urine receptacle used for the present invention, urine discharged in a top sheet is absorbed through a large number of urine drainage pores formed on perforated urine absorbent sheet. Therefore, the percentage of urine collection increases and the amount of urine which remains in the urine receptacle is reduced. With the increase in the percentage of urine collection, a small capacity vacuum pump with a low suction force can drain urine from the urine absorbent material. Therefore, it is possible to drain urine from the urine receptacle without discomfort to the wearer, and the device can be compact and lightweight.

Since the device can be compact and lightweight, if it is used as a portable automatic urine disposal device, it will be most efficient. Furthermore, because the device is compact and lightweight and the vacuum pump does not unnecessarily absorb air, noise is minimal and urine can be quietly drained without bothering other patients in the room at night.

Furthermore, in the aforementioned embodiments, peripheral pieces provided on the support sheet prevent urine from leaking from the sides. Consequently, urine leaks from the sides can be doubly prevented by the peripheral pieces and the gathers formed on the outer sheet. However, it is obvious that the peripheral pieces are not necessarily provided on the support sheet in a practical use of the urine receptacle.

Moreover, in the aforementioned embodiments, the top sheet is hard-breathable and the support sheet is non-breathable. However, if the pressure in the urine drainage pores of the perforated urine absorbent sheet is made negative, it is obvious that the same effect can be expected when those sheets are slightly breathable.

The present invention can increase the percentage of urine collection by the urine receptacle and reduce the amount of urine which remains in the urine receptacle. Therefore, a small capacity vacuum pump can drain urine from the urine receptacle. As a result, it is possible to drain urine from the urine receptacle without discomfort to the wearer, and the device can be compact and lightweight.

Since the device can be compact and lightweight, if it is used as a portable automatic urine disposal device, it will be most efficient. Furthermore, because the device is compact and lightweight and the vacuum pump does not unnecessarily absorb air, noise is minimal and urine can be quietly drained without bothering other patients in the room at night.

**Claims**

1. An automatic urine disposal device for absorbing urine collected in a urine receptacle (1) by means of a vacuum pump (23) and directing it to a urine tank (21), said urine receptacle (1) comprising

    a top sheet (2) disposed on said urine receptacle's surface which comes in contact with the wearer's skin,

    a perforated urine absorbent sheet (3) on which a large number of urine drainage pores (3a) for absorbing urine discharged in said top sheet are formed,

    a support sheet (5) disposed on said top sheet's opposite side which does not come in contact with the wearer's skin, and

    a urine absorbing space forming sheet (4) which is disposed closer to the support sheet (5) than said perforated urine absorbent sheet (3), **characterized in that**

    said urine tank (21) is sealed,

    said support sheet (5) has a first urine drainage means (10),

    a second urine drainage means (11) for directing urine into said urine tank (21) is connected to said first urine drainage means (10), and

    said vacuum pump (23) vacuums the inside of said urine tank (21).

2. An automatic urine disposal device according to claim 1, **characterized in that** said second urine drainage means (11) is a tube, and said vacuum pump (23) absorbs air from said urine tank (21).

3. An automatic urine disposal device according to claim 1 or 2,
    **characterized in that**

    said urine receptacle (1) has a urine sensor (9) for detecting that urine has been discharged in said urine receptacle (1) and activating said vacuum pump (23),

    said support sheet (5) is liquid-impermeable and non-breathable, and

    said vacuum pump (23) decreases pressure in said urine tank (21) and draws urine from a urine drainage means formed on said support sheet to said urine tank.

4. An automatic urine disposal device according to any of the preceding claims, **characterized in that**

    said urine tank (21) has a lid (22) for sealing said urine tank,

    said second urine drainage means (11) directs urine from a first urine drainage means (10) formed on said support sheet (5) to said urine tank via said lid,

    said vacuum pump (23) makes pressure of the urine drainage pores of said perforated urine absorbent sheet (3) negative and absorbs urine into said urine tank.

5. An automatic urine disposal device according to any of the preceding claims, **characterized in that**

    said top sheet (2) is liquid-permeable and hard-breathable,

    said urine absorbing space forming sheet (4) is fibrous, and

    said first urine drainage means (10) is connected to said second urine drainage means (11) by a joint (13).

6. An automatic urine disposal device according to any of the preceding claims, **characterized in that**

    said top sheet (2) covers the surface of said perforated urine absorbent sheet (3),

    said urine absorbing space forming sheet (4) is disposed between the bottom surface of said perforated urine absorbent sheet (3) and said support sheet (5).

7. An automatic urine disposal device according to any of the preceding claims, **characterized in that**

    said top sheet (2) is hard-breathable, and

    said support sheet (5) is liquid-impermeable.

8. An automatic urine disposal device according to any of the preceding claims, **characterized in that**
   said top sheet (2) is liquid-permeable and hard-breathable and covers the surface of said perforated urine absorbent sheet (3),
   said urine absorbing space forming sheet (4) is disposed such that it comes in close contact with the bottom surface of said perforated urine absorbent sheet (3) thereby forming a urine absorbing space,
   said support sheet (5) is liquid-impermeable and supports said urine absorbing space forming sheet (4), and
   said urine receptacle has an outer sheet (7) which holds the aforementioned sheets.

9. A urine receptacle (1) used for an automatic urine disposal device to absorb urine discharged from a wearer's urinating part, said urine receptacle comprising
   a perforated urine absorbent sheet (3) for absorbing discharged urine,
   a top sheet (2) which covers the surface of said perforated urine absorbent sheet (3),
   a urine absorbing space forming sheet (4) which is disposed such that it comes in close contact with the bottom surface of said perforated urine absorbent sheet (3), and
   a liquid-impermeable support sheet (5) which supports said urine absorbing space forming sheet and has a urine drainage means.

10. A urine receptacle (1) according to claim 9, **characterized in that**
    said perforated urine absorbent sheet (3) and said support sheet (5) are liquid-impermeable, and
    said top sheet (2) is hard-breathable.

11. A urine receptacle (1) according to claim 9 or 10, **characterized in that**
    said perforated urine absorbent sheet (3)and said support sheet (5) are liquid-impermeable and non-breathable, and
    said top sheet (2) is liquid-permeable and hard-breathable.

12. A urine receptacle according to any of claims 9 - 11, **characterized in that**
    said urine absorbing space forming sheet (4) is made of a fibrous material, and
    an outer sheet (7) is provided to support said top sheet, said perforated urine absorbent sheet, said urine absorbing space forming sheet and said support sheet.

13. A urine receptacle according to any of claims 9 - 12, **characterized in that**
    gathers are provided along the periphery of said support sheet.

14. A urine receptacle according to any of claims 9 - 13, **characterized in that**
    said urine absorbing space forming sheet (4) fits into the concave portion of said support sheet (5),
    said outer sheet (7) contains said top sheet (2), said perforated urine absorbent sheet (3), said urine absorbing space forming sheet (4) and said support sheet (5), and
    said urine absorbent sheet (6) is disposed between said support sheet (5) and said outer sheet (7).

# FIG. 1

## FIG. 2

## FIG. 3

# FIG. 4

## FIG. 5

## FIG. 6

## FIG. 7

## FIG. 8

(a)

(b)

**European Patent Office**

**EUROPEAN SEARCH REPORT**

Application Number

EP 04 01 3652

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 5 911 222 A (LAWRENCE W THOMPSON ET AL) 15 June 1999 (1999-06-15) * column 5, line 24 - column 6, line 42 * * column 7, line 48 - line 57 * * column 8, line 60 - column 9, line 40 * | 1-7,9 | A61F5/455 A61F5/44 A61F13/15 |
| Y | * figures 2A,6 * --- | 8,10-13 | |
| X | WO 93/09736 A (KUNTZ DAVID H ;ELSON EDWARD E (US)) 27 May 1993 (1993-05-27) * page 9, line 27 - page 11, line 22 * * page 13, line 1 - page 14, line 7 * * page 14, line 17 - line 21 * * figures 1,3 * --- | 1,2,4,6, 7,9 | |
| Y | GB 2 244 653 A (KAO CORP) 11 December 1991 (1991-12-11) * page 5, paragraph 1 - paragraph 2 * * page 12, paragraph 4 - page 13, paragraph 1 * * figures 1,2,6 * --- | 8,10-13 | |
| A | EP 0 312 118 A (KIMBERLY CLARK CO) 19 April 1989 (1989-04-19) * page 3, line 17 - line 33 * * page 4, line 3 - line 6 * ----- | 1,9 | TECHNICAL FIELDS SEARCHED (Int.Cl.7) A61F |

The present search report has been drawn up for all claims

| Place of search | Date of completion of the search | Examiner |
|---|---|---|
| MUNICH | 18 August 2004 | Storer, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 04 01 3652

This annex lists the patent family members relating to the patent documents cited in the above-mentioned European search report.
The members are as contained in the European Patent Office EDP file on
The European Patent Office is in no way liable for these particulars which are merely given for the purpose of information.

18-08-2004

| Patent document cited in search report | | Publication date | Patent family member(s) | | Publication date |
|---|---|---|---|---|---|
| US 5911222 | A | 15-06-1999 | US 5678564 A | | 21-10-1997 |
| | | | AT 226421 T | | 15-11-2002 |
| | | | AU 679936 B2 | | 17-07-1997 |
| | | | AU 4997593 A | | 03-03-1994 |
| | | | CA 2141855 A1 | | 17-02-1994 |
| | | | DE 69332436 D1 | | 28-11-2002 |
| | | | DE 69332436 T2 | | 10-07-2003 |
| | | | DK 653928 T3 | | 24-02-2003 |
| | | | EP 0653928 A1 | | 24-05-1995 |
| | | | ES 2187510 T3 | | 16-06-2003 |
| | | | JP 8510924 T | | 19-11-1996 |
| | | | NO 950422 A | | 29-03-1995 |
| | | | NZ 255677 A | | 26-05-1997 |
| | | | WO 9403214 A2 | | 17-02-1994 |
| | | | ZA 9305721 A | | 07-06-1994 |
| WO 9309736 | A | 27-05-1993 | AU 665008 B2 | | 14-12-1995 |
| | | | AU 2146892 A | | 15-06-1993 |
| | | | BR 9206795 A | | 31-10-1995 |
| | | | CA 2122137 A1 | | 27-05-1993 |
| | | | DE 69217078 D1 | | 06-03-1997 |
| | | | DE 69217078 T2 | | 07-05-1997 |
| | | | EP 0613355 A1 | | 07-09-1994 |
| | | | JP 8505061 T | | 04-06-1996 |
| | | | WO 9309736 A2 | | 27-05-1993 |
| GB 2244653 | A | 11-12-1991 | HK 75895 A | | 26-05-1995 |
| | | | JP 4088932 U | | 03-08-1992 |
| | | | SG 9590700 A2 | | 01-09-1995 |
| EP 0312118 | A | 19-04-1989 | US 4798603 A | | 17-01-1989 |
| | | | AT 134128 T | | 15-02-1996 |
| | | | AU 599839 B2 | | 26-07-1990 |
| | | | AU 2367088 A | | 20-04-1989 |
| | | | BR 8805332 A | | 30-05-1989 |
| | | | CA 1312175 C | | 05-01-1993 |
| | | | DE 3855005 D1 | | 28-03-1996 |
| | | | DE 3855005 T2 | | 13-06-1996 |
| | | | DE 3856448 D1 | | 08-02-2001 |
| | | | DE 3856448 T2 | | 23-08-2001 |
| | | | DE 8817262 U1 | | 21-03-1996 |
| | | | EP 1046383 A2 | | 25-10-2000 |
| | | | EP 0312118 A2 | | 19-04-1989 |
| | | | EP 0682930 A1 | | 22-11-1995 |
| | | | ES 2085850 T3 | | 16-06-1996 |
| | | | ES 2153001 T3 | | 16-02-2001 |

EPO FORM P0459

For more details about this annex : see Official Journal of the European Patent Office, No. 12/82

EP 1 486 184 A1

## ANNEX TO THE EUROPEAN SEARCH REPORT
## ON EUROPEAN PATENT APPLICATION NO.

EP 04 01 3652

This annex lists the patent family members relating to the patent documents cited in the above-mentioned European search report.
The members are as contained in the European Patent Office EDP file on
The European Patent Office is in no way liable for these particulars which are merely given for the purpose of information.

18-08-2004

| Patent document cited in search report | Publication date | Patent family member(s) | | Publication date |
|---|---|---|---|---|
| EP 0312118        A | | HK | 1006394 A1 | 26-02-1999 |
| | | JP | 2001266 A | 05-01-1990 |
| | | JP | 2818796 B2 | 30-10-1998 |
| | | KR | 9707415 B1 | 08-05-1997 |
| | | KR | 9707413 B1 | 08-05-1997 |
| | | MX | 162782 A | 26-06-1991 |
| | | ZA | 8807687 A | 30-05-1990 |

EPO FORM P0459

For more details about this annex : see Official Journal of the European Patent Office, No. 12/82

15